**Europäisches Patentamt**

**European Patent Office**

**Office européen des brevets**

(19)

(11) Publication number: **0 077 681**
**B1**

## (12) EUROPEAN PATENT SPECIFICATION

(45) Date of publication of patent specification: **05.11.86**

(21) Application number: **82305536.3**

(22) Date of filing: **18.10.82**

(51) Int. Cl.⁴: **A 61 B 17/58**

(54) Bone fixation screws.

(30) Priority: **16.10.81 GB 8131245**

(43) Date of publication of application:
**27.04.83 Bulletin 83/17**

(45) Publication of the grant of the patent:
**05.11.86 Bulletin 86/45**

(84) Designated Contracting States:
**AT BE CH DE FR IT LI LU NL SE**

(56) References cited:
**FR-A-1 037 262**
**FR-A-2 381 511**
**US-A-3 530 584**
**US-A-3 554 193**

(73) Proprietor: **D. HOWSE & COMPANY LIMITED**
**29 Beethoven Street**
**London W10 4LR (GB)**

(72) Inventor: **Howse, Derek Leslie**
**1 Kitchers Close Sway**
**Lymington Hampshire (GB)**

(74) Representative: **Woodin, Anthony John et al**
**Fitzpatricks Kern House**
**61/62 Lincoln's Inn Fields**
**London WC2B 6EX (GB)**

Courier Press, Leamington Spa, England.

## Description

This invention is concerned with bone fixation screws, and particularly but not exclusively with screws for fixing appliances to the bones of children.

Children's bones are usually much softer than those of adults and surgeons, when attempting to tighten a screw into cancellous bone, may be faced with the difficulty that the thread of the screw fails to grip the soft cancellous bone so that the screw will not tighten and an unsatisfactory fixation results. It is an object of the present invention to provide means for overcoming or at least reducing this difficulty.

According to the invention, there is provided a bone fixation screw having a shaft provided with an enlarged wrench head at one end and a screw thread at the other end, and a projection from the head on the opposite side from the shaft, which projection is screw-threaded for carrying a nut, characterised in that a screw thread is also provided on the shaft under and adjacent to the head. Thereby, the second-said screw thread will in use engage with the hard cortex bone, enabling the surgeon to tighten the second-said screw thread into the hard bone and obtain a firmer fixation. The nut can be used for tightening up against the head a fixation plate carried by the projection.

Preferably the second-said screw thread is immediately under the head.

Preferably, the diameter of the screw thread under the head is greater than that of the first-said screw thread.

The screw is preferably cannulated, i.e. it has an axial through hole.

The screw threaded parts of the shaft are preferably separated by an unthreaded part, but may abut each other.

The invention further provides a screw of the aforementioned kind having a screw thread under and adjacent to the head in combination with a perforated fixation plate adapted to be received on the projection.

The following is a description, by way of example, of bone fixation screws in accordance with the invention, with reference to the accompanying drawings, in which

Fig. 1 is a side elevation of a conventional bone fixation screw,

Fig. 2 is a side elevation of a bone fixation screw in accordance with the invention, and

Fig. 3 is a sectional view through part of a bone showing the screw of Fig. 2 in use.

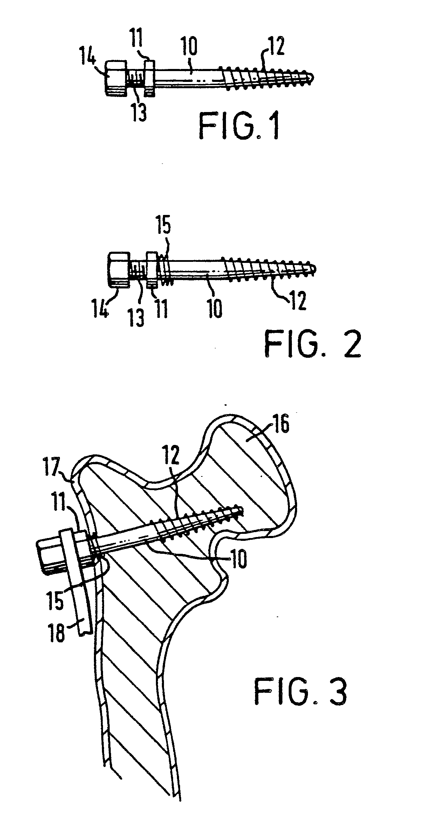

The screws shown in the drawings are in the form of lag screws and each comprises a shaft 10 with a nut-like head 11 at one end and a screw thread 12 at the other end. On the opposite side to the shaft, the head has a screw-threaded projection 13 carrying a nut 14. The screw has an axial through hole for passage over a guide wire when being inserted. The conventional screw shown in Fig. 1 has an unthreaded part extending from under the head 11 to the screw thread 12. In the screw according to this invention shown in Fig. 2, a screw thread 15 is provided on the shaft 10 under and immediately adjacent to the head 11. The screw thread 15 is of greater diameter than the screw thread 12. As can be seen from Fig. 3, in use the screw thread 12 engages in soft cancellous bone 16 while the screw thread 15 engages in hard cortex bone. After the screw has been inserted into the bone, a fixation plate 18 (of which only part is shown in Fig. 3) is passed over the projection 13 with the nut 14 removed, the projection passing through a hole in the plate. The nut 14 is then screwed on to projection 13 so as to clamp the plate 18 against the head 11. The other part of the plate is of course perforated and secured by screws in known manner to another part of the bone.

The screw thread 15 extends over a smaller part of the length of the shaft than the screw thread 12.

## Claims

1. A bone fixation screw having a shaft (10) provided with an enlarged wrench head (11) at one end and a screw thread (12) at the other end, and a projection (13) from the head on the opposite side from the shaft, which projection is screw-threaded for carrying a nut (14), characterised in that a screw thread (15) is also provided on the shaft under and adjacent to the head.

2. A bone fixation screw according to claim 1 wherein the second-said screw thread (15) is immediately under the head.

3. A bone fixation screw according to claim 1 or claim 2 wherein the diameter of the screw thread (15) under the head is greater than that of the first-said screw thread (12).

4. A bone fixation screw according to any preceding claim which is cannulated.

5. A bone fixation screw according to any preceding claim in combination with a perforated fixation plate (18) adapted to be received on the projection (13).

6. A bone fixation screw according to any preceding claim wherein the shaft (10) has an unthreaded part separating said screw threads (12, 15).

7. A bone fixation screw according to claim 6 wherein the screw thread (15) under the head extends over a smaller part of the length of the shaft than the first-said screw thread (12).

## Patentansprüche

1. Schraube zur Knochenbefestigung mit einem Schaft (10) mit einem vergrößerten Schraubkopf (11) an einem Ende und einem Schraubgewinde (12) am anderen Ende sowie mit einem Ansatz (13) am Kopf auf der gegenüberliegenden Seite des Schafts, wobei besagter Ansatz eine Gewinde für eine Mutter (14) aufweist, dadurch gekennzeichnet, daß auch auf dem Schraubenschaft unter und bis angrenzend an den Schraubkopf eine Gewinde (15) angeordnet ist.

2. Schraube zur Knochenbefestigung gemäß Anspruch 1 bei der sich das besagte zweite Schraubgewinde (15) direkt unter dem Schraubkopf befindet.

3. Schraube zur Knochenbefestigung gemäß Anspruch 1 oder Anspruch 2, bei der der Durchmesser des Schraubgewindes (15) unter dem Schraubkopf größer ist als der des besagten ersten Schraubgewindes (12).

4. Schraube zur Knochenbefestigung gemäß einem beliebigen der vorhergehenden Ansprüche, die kanulliert ist.

5. Schraube zur Knochenbefestigung gemäß einem beliebigen der vorhergehenden Ansprüche kombiniert mit einer perforierten Befestigungsplatte (18), die so ausgebildet ist, daß sie auf den Ansatz (13) paßt.

6. Schraube zur Knochenbefestigung gemäß einem beliebigen der vorhergehenden Ansprüche bei der der Schraubenschaft (10) einen die beiden Schraubgewinde (12, 15) trennenden Teil ohne Gewinde aufweist.

7. Schraube zur Knochenbefestigung gemäß Anspruch 6 bei der sich das Schraubgewinde (15) unter dem Schraubkopf über einen kleineren Teil der Länge des Schafts erstreckt als das erste besagte Schraubgewinde (12).

**Revendications**

1. Une vis de fixation des os munie d'un arbre (10) portant une tête clavetable élargie (11) à une extrémité et un filet de vissage (12) à l'autre, et une projection (13) en saillie de la tête sur le côté opposé de l'arbre, cette projection étant filetée pour accepter un écrou (14), caractérisée en ce qu'un filet de vissage (15) est également prévu sur l'arbre en-dessous de la tête et en position adjacente à celle-ci.

2. Une vis de fixation des os conforme à la revendication 1, sur laquelle le deuxième filet de vissage précité (15) est directement sous la tête.

3. Une vis de fixation des os conforme à la revendication 1 ou la revendication 2, sur laquelle le diamètre du filetage (15) sous la tête est supérieur à celui du premier filetage précité (12).

4. Une vis de fixation des os conforme à l'une quelconque des revendications qui précèdent et qui est canalisée.

5. Une vis de fixation des os conforme à l'une quelconque des revendications qui précèdent combinée avec une plaque de fixation (18) perforée et adaptée pour s'introduire sur ladite projection (13).

6. Une vis de fixation des os conforme à l'une quelconque des revendications qui précèdent sur laquelle l'arbre (10) possède une partie non filetée qui sépare les filets de vissage précités (12, 15).

7. Une vis de fixation des os conforme à la revendication 6 sur laquelle le filet de vissage (15) sous la tête s'allonge sur une partie plus petite de la longueur entière de l'arbre que le premier filet de vissage précité (12).

FIG.1

FIG. 2

FIG. 3